# EUROPÄISCHE PATENTSCHRIFT

(11) **EP 0 774 262 B1**
(45) Veröffentlichungstag und Bekanntmachung des Hinweises auf die Patenterteilung: **30.05.2001**
(21) Anmeldenummer: 96113398.0
(22) Anmeldetag: 21.08.1996
(51) Int. Cl.: A61K 39/42

(54) **Pharmazeutische Zusammensetzungen enthaltend ein neutralisiertes Virus, und Verwendung derselben**
Pharmaceutical compositions comprising a neutralized virus and use thereof
Compositions pharmaceutiques comprenant un virus neutralisé et leur utilisation

(30) Priorität: 24.08.1995 DE 19531226
(43) Veröffentlichungstag der Anmeldung: 21.05.1997
(73) Patentinhaber: Baxter Aktiengesellschaft, 1221 Wien (AT)
(72) Erfinder: Eder, Gerald, Dr., 3031 Rekawinkel (AT); Eibl, Johann, Dr., 1180 Wien (AT)
(74) Vertreter: Kolb, Helga, Dr. Dipl.-Chem.

(56) Entgegenhaltungen:
- WO-A-91/04750
- THE JOURNAL OF INFECTIOUS DISEASES, Bd. 165, 1992, Seiten 438-443, XP000651120 PRINCE, A.M. ET AL.: "Immunity in Hepatitis C Infections"
- THE JOURNAL OF GENERAL VIROLOGY, Bd. 69, 1988, Seiten 2505-2516, XP000651200 RANDALL R.E AND D.F. YOUNG: "Humoral and Cytotoxic T Cell Immune Responses to Internal and External Structural Proteins of Simian Virus 5 Induced by Immunization with Solid Matrix-Antibody-Antigen Complexes"

## Beschreibung

Die vorliegende Erfindung betrifft pharmazeutische Zusammensetzungen, welche die Kombination aus einem Virus mit einem neutralisierenden Antikörper enthalten, sowie die Verwendung derselben.

Die Erkenntnis, dass eine vorherige Exposition gegenüber kleiner Mengen eines Krankheitserregers oder einem Fragment davon einen nachfolgenden Schutz vor einer ernsthaften Erkrankung bieten kann, hat es ermöglicht, bedeutende Viruserkrankungen, wie Pocken, Gelbfieber, Tollwut, Kinderlähmung, Masern und Mumps zu verhüten. Insbesondere im Hinblick auf den Mangel antiviraler Medikamente zeitigten die gegen Viren gerichteten Impfungen dramatische Erfolge.

Zur Entwicklung eines Impfstoffes stehen mehrere klar definierte Wege zur Verfügung. Man kann andere, zum Zielvirus verwandte Viren verwenden, die zwar das Abwehrsystem stimulieren, aber selbst keine ernsthafte Erkrankung nach sich ziehen. Diesen Weg ist man z.B. bei der Bekämpfung der Pocken- oder Kuhpocken-Erreger gegangen. Es lassen sich aber auch Viren künstlich so abwandeln, dass ihnen zwar ihre Fähigkeit, eine ernsthafte Krankheit zu verursachen, nicht aber ihr zur Stimulierung des Abwehrsystems erforderlicher Antigenbestand abhanden gekommen ist. Solche attenuierten (abgeschwächten) lebenden Viren werden gegenwärtig in den Impfstoffen gegen Masern, Polio, Mumps und Röteln eingesetzt.

Ein weitere Weg bietet sich im 'Abtöten' des entsprechenden Virus vor seiner Anwendung als Impfstoff. Hier müssen die erforderlichen Antigene trotz des Verlustes der Fortpflanzungsfähigkeit und der Pathogenität intakt bleiben. Der Nachteil dieser Totimpfstoffe gegenüber Lebendimpfstoffen liegt darin, dass ihre Immunisierung schwächer ausfällt und sie deshalb in regelmässigen Abständen verabreicht werden müssen, um einen wirksamen Schutz zu gewährleisten.

Wenn man davon ausgeht, dass allein die Präsenz viraler Antigene, die sich auf Fragmenten des Virus befinden, über den Impferfolg entscheidet, so ist verständlich, dass Impfstoffe auch aus Virusfragmenten oder gereinigten viralen Proteinen hergestellt werden können. Letztere Methode wird vor allem für die Zukunft breite Anwendung finden, da es heute möglich ist, gentechnologisch die gewünschten Antigene in einfacher Weise und in ausreichender Menge herzustellen. Diese Methode erprobt man derzeit, um einen Schutz gegenüber Hepatitis-, Herpes- und AIDS-Viren zu erzielen.

Unabhängig von der Methode der Impfstoffherstellung sind die erwünschten Eigenschaften immer die gleichen. Der Impfstoff muss selbstverständlich das Abwehrsystem wirksam stimulieren, um auf diese Weise eine möglichst langlebige Immunität zu hinterlassen. Ausserdem dürfen die Impfstoffe keine gravierenden Nebenwirkungen aufweisen; und für einen weltweiten Einsatz soll der Impfstoff billig und über lange Zeitperioden stabil sein.

Eine Schwierigkeit bei der modernen Impfstoffherstellung besteht darin, dass durch eine hohe Mutationsrate der Viren eine Reihe von Impfstoffen nur eine ungenügende Immunität hervorruft. In solchen Fällen ist es erforderlich, nach Methoden der Impfstoffgewinnung zu suchen, die beim Wirt eine höhere Immunität hervorrufen und diesen somit vor dem Krankheitserreger wirksam zu schützen vermögen.

Es ist im Stand der Technik bekannt, dass wenn man Schimpansen mit einer infektiösen Faktor VIII-Präparation inokuliert und dazu gleichzeitig eine menschliche Immunglobulin-Präparation verabreicht, diese Schimpansen eine akute nicht-A, nicht-B Hepatitis entwickeln. Die Verwendung von Immunglobulin kann also in diesem Falle die Infektion nicht verhindern (Spichtin H.-P. et al, Journal of Medical Virology 12, 215-226 (1983)).

Die Fortführung der vorstehenden Arbeiten wurde von Eder et al (Viral Hepatitis and Liver Disease, 550-552; 1988) beschrieben: Eine erneute Inokulation der Versuchstiere nach 2 und 4 Jahren ergab, dass diese wiederum an nicht-A, nicht-B Hepatitis erkrankten.

Von der gleichen Forschergruppe wurde im Jahre 1990 (Clin. exp. Immunol. 81, 454-458, 1990) festgestellt, dass multiple Infusionen von i.v. Immunglobulin in Schimpansen nicht zu einer Immuneliminierung führen.

Arbeiten von Prince A.M. et al (The Journal of Infectious Diseases, 165:438-43; 1992) beschreiben, dass im Falle einer erneuten Inokulierung von Schimpansen mit HCV 59% der Versuchstiere eine Serokonversion zeigten, 33% HCV-assoziierte hepatozelluläre ultrastrukturelle Veränderungen aufwiesen und 26% eine Hepatitis C entwickelten. Insgesamt zeigten 74% der Versuchstiere nach Reinfektion mit HCV mindestens eines der vorstehend genannten Symptome.

Von Farci P. et al (Science, 250, 135-140; 1992) wird in der Publikation 'Lack of Protective Immunity Against Reinfection with Hepatitis C Virus' beschrieben, dass Versuche an 5 Schimpansen gezeigt haben, dass eine HCV-Infektion keine protektive Immunität gegen eine Reinfektion mit homologen oder heterologen Stämmen hervorruft. Die Autoren zeigten daher Zweifel in bezug auf die Entwicklung wirksamer Impfstoffe gegen HCV.

Im Stand der Technik ist auch die alleinige Verabreichung von Immunglobulin-Präparaten bekannt, und zwar insbesondere zur Substitutionsbehandlung von primären und sekundären Antikörper-Mangelzuständen, wie z.B. die Behandlung von Autoimmun- und Immunkomplexerkrankungen, wie der idiopathischen thrombocytopenischen Purpura, die Adjuvanstherapie bakterieller Infektionen, insbesondere bei akuten bakteriellen Infektionen, wie Sepsis, Meningitis, Peritonitis oder bei chronisch bakteriellen Infektionen, wie Ostiomyelitis. In diesen Fällen erfolgt die Verabreichung intravenös. Es ist ausserdem bekannt, dass intravenöses Gammaglobulin (Endobulin® ) zur Sofortprophylaxe von Viruserkrankungen eingesetzt werden kann.

In der Literatur werden Versuche zur Wirkung neutralisierender Antikörper gegen umhüllte Viren beschrieben (Nature, Vol. 321, 244-246 (1986)). Die Autoren Gollins et al beschreiben ein in vitro-Modell zur Erklärung des Wirkmechanismus eines neutralisierenden Antikörpers. Hierzu wurden in vitro-Versuche mit Immunglobulin-konditionierten P388D1-Zellen und Kaninchen-IgG durchgeführt. Es konnte gezeigt werden, dass durch die Zugabe von Immunglobulin bei 37°C eine dosisabhängige Inhibierung der Entfernung ("uncoating") der Virushülle und damit der Infektion der Zelle verhindert wird. Auf Seite 245, rechte Spalte, letzter Absatz, wird ausgeführt, daß anzunehmen ist, daß aufgrund der Anwesenheit neutralisierender Antikörper eine Fusion zwischen Viruspartikel und endosomaler Membran unterbunden wird. Aus dieser Kenntnis leiteten die Autoren ab, wie zukünftig neutralisierende Antikörper optimal konstruiert sein sollten. Die Verabreichung eines Komplexes oder Konjugats aus Antigen und neutralisierendem Antikörper wird in der vorstehenden Literatur nicht beschrieben, außer WO 91/04750.

Im Stand der Technik ist auch die sogenannte Simultanimpfung bekannt, bei der parallel, d.h. separat, jeweils ein viraler Impfstoff zur passiven und aktiven Immunisierung verabreicht wird. Sobald ein Patient einem Infektionsrisiko ausgesetzt ist, werden zur Simultanimpfung üblicherweise ein Hyperimmunglobulin-Präparat zur unmittelbaren Neutralisierung des Krankheitserregers und ein entsprechendes Antigen zur Stimulierung der körpereigenen Antikörperproduktion verabreicht. Beispielsweise wird post-expositionell ein Immunglobulin-Präparat spezifisch für das FSME-Virus (TBE-Virus des westlichen Subtyps) und parallel dazu eine FSME-Vakzine auf Basis des inaktivierten Virus eingesetzt (Vaccine, Vol, 13(6), 759-762, 1995).

Aufgabe der vorliegenden Erfindung ist es, eine pharmazeutische Zusammensetzung zur Verfügung zu stellen, welche einen verbesserten Impfstoff darstellt. Insbesondere soll die pharmazeutische Zusammensetzung eine verstärkte Immunität bei Menschen und Tieren hervorrufen, um auf diese Weise bei stark mutierenden Viren einen ausreichenden Schutz gegenüber dem Krankheitserreger zu schaffen.

Die vorstehende Aufgabe wird erfindungsgemäss gelöst durch eine pharmazeutische Zusammensetzung zur Prophylaxe bzw. Therapie von Viruserkrankungen, die verursacht sind durch Viren hoher Mutationsrate oder Viren, die in zahlreichen Gentyopen bzw. Subtypen existieren, wobei die Zusammensetzung ein Konjugat bzw. einen Komplex aus einem inaktivierten und angereicherten bzw. gereinigten Virus und neutralisierenden Antikörpern enthält. Mit anderen Worten bedeutet dies, dass zum Zwecke der Immunisierung das gegebenenfalls krankheitserregende Virus neutralisiert mit Antikörper verabreicht wird.

Die vorliegende Erfindung richtet sich allgemein auf die Behandlung von Säugern, d.h. auf die Behandlung von Mensch und Tier.

Als Zielvirus sind vor allem Viren mit hoher Mutationsrate von besonderer Bedeutung. Es gibt noch keinen Impfstoff zur allgemeinen Prophylaxe bzw. Therapie gegen eine virale Erkrankung, die durch verschiedenste Virusmutanten hervorgerufen wird. Sobald Antikörper gegen einen Infektionserreger gebildet werden, besteht bei diesen Viren bereits das Risiko der weiteren Veränderung des Virus, wodurch die vorhandenen Antikörper nicht mehr neutralisierend wirken können. Viren mit hoher Mutationsrate haben insbesondere die Eigenschaft, daß sie in veränderter Form einen Organismus immer wieder infizieren können. Diese sogenannte Reinfektion wird vor allem bei Viren beobachtet, die in den verschiedensten Erscheinungsformen, wie Quasispezien, Genotypen, Subtypen, und dergleichen auftreten.

Im speziellen ist das Zielvirus gemäss der Erfindung eines der von durch Blut übertragbaren Viren ("blood borne"). Diese Viren können aus Körperflüssigkeiten, wie Blut, Serum oder Plasma isoliert werden. Beispielhaft sind dabei die Hepatitiden zu nennen, darunter HAV, HBV und HCV, sowie die sogenannten Aids-Viren, wie HIV-1, aber auch Parvovirus, Cytomegalovirus, Epstein-Barr-Virus.

Neben den aus einer Körperflüssigkeit isolierbaren Viren, die sich teilweise durch lange Inkubationszeit und/oder durch relativ lange Virämie (Ventildauer der Viren in der Zirkulation) auszeichnen, sind vor allem humanpathogene Viren zu nennen, die immer wieder in den verschiedensten Erscheinungsformen auftreten.

Bedingt durch deren Variabilität treten beispielsweise verschiedene Influenza-A Viren auf, deren Infektion nur durch Impfstoffe auf Basis der entsprechenden inaktivierten Typen verhindert werden kann. Durch die erfindungsgemäße Präparation auf Basis eines neutralisierten Influenza-A Virus wird es erstmals möglich, eine Art der Präparation über Jahre hinweg global einzusetzen, unabhängig von den jeweils auftretenden Stämmen. Die Stabilität der Präparation macht es auch möglich, diese über einen Zeitraum von mindestens 2 Jahren zu lagern. In der erfindungsgemäßen Zusammensetzung sind vor allem ein Influenza-Virus oder eine bestimmte Mischung von Influenza-Viren enthalten, die in aktivierter Form vorliegen. Die Aktivierung des Virus wird beispielsweise durch eine Enzymbehandlung, insbesondere mit Trypsin, vorgenommen. Dadurch kommen auch die entsprechenden Enzyminhibitoren, beispielsweise Trypsininhibitor, zum Einsatz, wobei die Inhibitoren im Zuge der Reinigung der Viren abgereichert bzw. abgetrennt werden.

Zu diesem Zwecke wird gereinigtes bzw. angereichertes Virus mit geeigneten (spezifischen) Antikörpern so behandelt, dass das Virus nicht nur neutralisiert ist, sondern darüber hinaus in vivo zu keiner produktiven Infektion führt. Unter einer produktiven Infektion versteht man dabei, dass es nicht zu einer Erkrankung kommt, wohl aber Viren produziert werden.

Die erfindungsgemässe pharmazeutische Zusammensetzung ist insbesondere als Impfstoff zur Prophylaxe gegen eine Viruserkrankung verwendbar. Die prophylaktische Wirkung gemäss der Erfindung ist in ihrem Ausmass im Hinblick auf die im Stand der Technik gekannte Simultanimpfung überraschend.

Bisher war es im Stand der Technik nicht bekannt, ein Konjugat bzw. ein Komplex aus einem angereicherten bzw. gereinigten Virus und neutralisierenden Antikörpern erfolgreich zur Prophylaxe bzw. Therapie von Viruserkrankungen, die verursacht sind durch Viren hoher Mutationsrate oder Viren, die in zahlreichen Genotypen bzw. Subtypen existieren, erfolgreich anzuwenden.Wie aus der vorstehend genannten Publikation von Eder et al (1988) zur Infektion von Schimpansen mit einer HCV-infizierten Faktor VIII-Präparation hervorgeht, konnte die gleichzeitige Verabreichung (parallel oder simultan) mit Immunglobulinen die Infektion mit HCV und die Erkrankung der Schimpansen primär nicht verhindern. Bei einem neuerlichen Infektionsversuch, bei welchem gemäss der Erfindung gereinigtes HCV anstelle von HCV-infiziertem Material eingesetzt wurde, konnte die Infektion bei dem Versuchstier im Hinblick auf eine Reinfektion moduliert werden. In diesem Falle zeigte das Versuchstier keine morphologischen und biochemischen Anzeichen einer viralen Hepatitis. Von Interesse ist dabei, dass das verwendete Immunglobulin-Präparat Antikörper gegen das HCV-Core- und NS3-Protein enthielt, d.h. auch Antikörper gegen Nicht-Strukturkomponenten des Virus. Die dabei verabreichte Dosis war mit 100 mg/kg sehr hoch, und es kann davon ausgegangen werden, dass bis zu 500 mg IgG/kg verabreicht werden können.

Im erfindungsgemässen Präparat liegt das Virus zusammen mit den neutralisierenden Antikörpern insbesondere als Komplex bzw. als Konjugat vor. Die Assoziation der Komponenten besteht aufgrund der Immunaffinität, kann jedoch auch als kovalente Bindung, gegebenenfalls durch ein Bindeglied ("linker") ausgebildet sein. Diese ist beispielsweise auch durch eine chemische Vernetzungsreaktion oder durch die Ausbildung von Schwefelbrücken möglich.

Die Komplexbindung kann aber auch aufgrund von elektrostatischen, hydrophoben oder van der Waals schen Kräften bestehen. In einer weiteren bevorzugten Ausführungsform sind das Antigen und der Zellbestandteil an einem festen Träger adsorbiert bzw. co-adsorbiert. Der feste Träger kann beispielsweise auch eine Lipidkomponente, wie ein Liposom oder Phospholipidvesikel, sein. Dabei ist es von besonderem Vorteil, ein Adjuvans als Adsorbens einzusetzen. Zu diesem Zweck eignet sich vor allem ein Adjuvans auf Basis von Metallsalzen. Im besonderen eignen sich Hydroxide von Aluminium, Eisen und Metallen der Übergangselemente. Diese Adjuvanzien stimmulieren nicht nur die Immunantwort, sondern stabilisieren auch den Immunkomplex. Ein stabiler Immunkomplex ist vor allem dann erforderlich, wenn das neutralisierte Virus unter Umständen durch die Abtrennung der neutralisierenden Antikörper wieder aktiv und möglicherweise pathogen werden kann. Der Zusatz von Stabilisatoren ermöglicht die Verlängerung der Halbwertszeit in vitro und in vivo, wobei gewährleistet ist, daß kein ungebundenes Virus nach Verabreichung der erfindungsgemäßen pharmazeutischen Präparation vorliegt.

Die Stabilität der erfindungsgemäßen pharmazeutischen Präparation kann beispielsweise mit einem Inkubationsversuch getestet werden, der die Inkubation der erfindungsgemäßen Präparation unter definierten Bedingungen in Plasma oder einem entsprechenden geeigneten Medium vorsieht. Beispielsweise kann die Präparation bei einer Temperatur um 37° C für eine Dauer von bis zu mehreren Tagen inkubiert werden und die mögliche Dissoziation des Immunkomplexes durch die Abtrennung desselben bzw. die Bestimmung der gegebenenfalls separierten, d. h. ungebunden und gegebenenfalls wieder aktiven Viren bzw. Antikörper erfaßt werden.

Als weitere Adjuvanzien, die vorteilhafterweise auch eine stabilisierende Wirkung zeigen, sind auch anorganische Substanzen, wie diverse Aluminium- oder Eisenverbindungen, darunter die entsprechenden Hydroxide und Phosphate, Öle, Phospholipide, insbesondere in vesikulärer Form, Polypeptide oder Zytokine zu nennen.

Die erfindungsgemässen Zusammensetzungen enthalten bevorzugt Flaviviren oder Pestiviren. Nach einer besonders bevorzugten Ausführungsform, die aber nicht als beschränkend zu sehen ist, enthalten die erfindungsgemässen Zusammensetzungen Hepatitis C-Virus.

Prinzipiell aber kommen in den erfindungsgemässen pharmazeutischen Zusammensetzungen DNA- und RNA-Viren zur Anwendung. Dabei sind als für den Menschen wichtige DNA-Viren die Familien der Parvoviren, Papovaviren, Hepadnaviren, Adenoviren, Herpesviren und Poxviren zu nennen. Bei diesen Familien sind von besonderer Bedeutung: Parvovirus B19, Papovavirus JC, BK, SV40, Papillomavirus, Hepatitis B-Virus, Adenovirus, Herpes Simplex 1 und 2-Virus, Varicella Zoster, EBV, CMV und Herpes 6-Virus.

Unter den für den Menschen wichtigen RNA-Viren und die von ihnen verursachten Erkrankungen sind insbesondere zu nennen: Röteln aus der Familie Togavirus, Hepatitis C aus der Familie Flavivirus, Hepatitis E aus der Familie Calcivirus, Hepatitis A und Coxackievirus aus der Familie Picornavirus oder die HTLV-I und II- sowie HIV-Viren aus der Gruppe der Retroviren. Als Beispiel der hüllenlosen Viren seien die Reoviren, z.B. Rotavirus oder California Tick-Fever-Virus, sowie die Hepatitis A (Familie der Picornaviren) oder Hepatitis E (Familie der Caliciviren) genannt.

Nach einer bevorzugten Ausführungsform enthalten die erfindungsgemässen pharmazeutischen Zusammensetzungen das neutralisierte Virus in gereinigter Form, insbesondere in hoch angereicherter oder reiner Form.

Die Verwendung von hochgereinigtem Virus hat darüber hinaus den Vorteil, dass eine Trennung in die verschiedenen Genotypen bzw. Subtypen erfolgen kann. Auf diese Weise kann es zu einer besseren Neutralisationswirkung des Antikörpers kommen.

Die Reinigung der Viren, die zur Herstellung der erfindungsgemäßen Zusammensetzung bereitgestellt werden, wird vorzugsweise durch Methoden der Chromatographie, insbesondere Affinitätschromatographie oder Gelfiltration, Filtration, beispielsweise mit Nanofilter, Adsorptionsbehandlungen, Präzipitation, beispielsweise Fällungsreaktionen mit Alkohol oder Tensiden, oder Zentrifugation, insbesondere Dichtegradienten-zentrifugation, vorgenommen. Damit werden vor allem kontaminierende Materialien des Ausgangsmaterials auf ein pharmazeutisch akzeptables Maß abgereichert.

Genauso wie das Virus aus einem Kulturmedium gereinigt wird, kann auch das neutralisierte Virus, welches als Konjugat bzw. als Komplex vorliegt, mit gleichen Methoden weiter gereinigt werden. Damit soll gewährleistet sein, daß nichtneutralisierte Viren abgetrennt werden.

Nach einer weiteren Ausführungsform gemäss der Erfindung handelt es sich bei dem Virus um ein rekombinant in Prokaryonten oder Eukaryonten hergestelltes Virus. Es ist ausserdem auch möglich, anstelle des Virus Fragmente desselben einzusetzen, welche geeignete Antigene aufweisen.

Ausserdem enthalten die pharmazeutischen Zusammensetzungen gemäss der Erfindung neben einem neutralisierten bzw. neutralisierbaren Virus inaktivierte Viren.

Die Erfindung sieht auch den Einsatz von ausschliesslich inaktivierten Viren vor. In diesem Fall wird die eingesetzte Virus-Fraktion zur Inaktivierung von Viren bevorzugt chemisch oder physikalisch behandelt.

Im Fall von inaktivierten Viren, die mit chemischen und/oder physikalischen Methoden behandelt sind, kann die Inaktivierung vor der Neutralisierung mit den Antikörpern oder am Immunkomplex bzw. Konjugat vorgenommen werden. Dafür eignet sich vor allem eine Hitzebehandlung, Strahlenbehandlung sowie eine Behandlung mit Chemikalien, wie Lösungsmitteln bzw. Detergenzien und chaotropen Substanzen.

Die für die Neutralisierung der erfindungsgemässen pharmazeutischen Zusammensetzungen verwendeten Antikörper werden bevorzugt aus Plasma oder einer Plasmafraktion gewonnen. Darüber hinaus ist es aber auch möglich, die Antikörper aus einer Zellkultur zu gewinnen.

Nach einer bevorzugten Ausführungsform können die Antikörper, die in der vorstehend beschriebenen Weise erhalten wurden, in einer Fraktion vorliegen, die zur Inaktivierung bzw. Abreicherung von möglicherweise vorhandenen Viren vorbehandelt ist.

Zur Herstellung einer erfindungsgemäßen Zusammensetzung eignen sich im Fall der von durch Blut übertragbaren Viren vor allem Antikörper, die aus einem Hyperimmunserum bzw. einem Serum von Patienten, die mit dem Krankheitserreger in Kontakt gekommen sind, gewonnen werden können. Bevorzugte Antikörper sind polyklonale Antikörper, insbesondere humane Antikörper, die monospezifisch oder polyspezifisch sein können. Bei monospezifischen Antikörpern ist eine Mischung einer Vielzahl von Antikörpern bevorzugt.

Es ist bevorzugt, dass die zur Neutralisierung des Virus in den erfindungsgemässen pharmazeutischen Zusammensetzungen verwendeten Antikörper gegen mehr als ein Epitop gerichtet sind. Besonders bevorzugt ist es, wenn die Antikörper gegen mehr als ein virales Antigen gerichtet sind.

Die zur Neutralisierung der erfindungsgemässen pharmazeutischen Zusammensetzungen eingesetzten Antikörper sind gegen DNA sowie RNA-Viren gerichtet. Es kann sich dabei um Viren mit einer Proteinhülle bzw. um nackte Viren handeln. Bevorzugt dabei ist es, dass die Antikörper gegen Kernproteine, d.h. Nicht-Strukturproteine und/oder gegen Hüllproteine gerichtet sind.

Jedenfalls ist es erforderlich, daß die ausgewählten Antikörper das Virus neutralisieren. Die Neutralisierung kann lediglich soweit gehen, daß das Virus zwar nicht mehr pathogen wirkt, jedoch den Wirt produktiv infizieren kann.

Dieser Fall der produktiven Infektion ist so definiert, daß das Virus sich repliziert und zumindest teilweise wieder Viruspartikel bildet, ohne daß der Wirt erkrankt. Im Fall der Neutralisierung des Virus, die die produktive Infektion erlaubt, ist es bevorzugt, einen Überschuß von Antikörpern in der erfindungsgemäßen pharmazeutischen Zusammensetzung einzusetzen. Dadurch wird gewährleistet, daß auch bei der Replikation des Virus in vivo die Virulenz nicht zu einer Erkrankung des Patienten führt.

Eine spezielle Ausführungsform bezieht sich auf die Neutralisierung des Virus, wobei jedoch auch die produktive Infektion unterbunden wird. Als bevorzugte Antikörper werden protektive Antikörper eingesetzt, die die Eigenschaft haben, einen Organismus vor einer Viruserkrankung zu schützen. Die Auswahl der Antikörper kann beispielsweise in einem Expositionsversuch ("challenge model") vorgenommen werden. Nach Exposition eines Modelltieres mit dem Pathogen kann beurteilt werden, ob die eingesetzten Antikörper vor dem Ausbruch der Krankheit oder deren Folgen schützen. Als Maßstab zählt beispielsweise die Veränderung von physiologischen Indikatoren, wie Antikörpertiter oder andere Blutwerte bzw. der Tod des Tieres.

Die erfindungsgemäß verwendeten Antikörper sind vorzugsweise native, intakte Antikörper, die im wesentlichen frei sind von Immunglobulinaggregaten. Damit wird eine unzulässig hohe spezifische antikomplementäre Aktivität verhindert. Es hat sich herausgestellt, daß Antikörperfragmente weniger geeignet sind, da der freie, also ungebundene Fc-Teil des Antikörpers wesentlich zur Immunantwort beiträgt.

Nach einer bevorzugten Ausführungsform kann die erfindungsgemässe Zusammensetzung weitere Substanzen umfassen, welche eine Verbesserung hinsichtlich der Neutralisierung des Virus oder eine Verstärkung der Immunantwort bewirken. So kann die erfindungsgemässe Zusammensetzung z.B. Komplement enthalten.

Gemäß einer bevorzugten Ausführungsform ist die erfindungsgemäße Zusammensetzung zur intravenösen Verabreichung geeignet. Damit hat diese Präparation Eigenschaften, die entsprechend den Richtlinien des Deutschen Arneimittelbuchs DAB 10 (3. Nachtrag 1994), "Immunglobulin vom Menschen zur intravenösen Anwendung", für intravenös verträgliche Immunglobulinpräparate definiert sind. Eine weitere Ausführungsform betrifft eine entsprechende Zusammensetzung, die oral verabreichbare bzw. mukosal aufnehmbare Antikörper enthält. Zu diesen zählen vor allem IgA-Antikörper, bei denen bekannt ist, daß sie über die Mukosa aufgenommen werden können.

Im weiteren sind auch jene Antikörper als mukosal verabreichbar definiert, die beispielsweise der Antikörperzusammensetzung aus Colostrum entsprechen.

Neben der intravenösen und oralen Verabreichungsmöglichkeit der erfindungsgemäßen Zusammensetzung sind weitere systemische oder lokale Wege der Verabreichung möglich. Unter anderem kann eine entsprechende Vakzine sowohl intramuskulär oder subkutan, rektal, nasal oder intracavitär verabreicht werden.

Im übrigen kann man sich bei der zu verabreichenden Dosis an IgG als oberen Wert an den Dosen orientieren, wie sie z.B. bei der Substitutionsbehandlung der primären und sekundären Antikörper-Mangelzustände gegeben werden. In diesen Fällen verabreicht man bis zu 1000 mg/kg Körpergewicht innerhalb von 14 Tagen. Mittlere Dosen dürften denen entsprechen, wie sie beim therapeutischen Plasmaaustausch eingesetzt werden: Hier werden in der Regel 200 mg/kg Körpergewicht verabreicht, wobei auch in diesem Fall die Dosis von intravenösem Gammaglobulin bis auf 1.000 mg/kg Körpergewicht/Tag gesteigert werden kann.

Untere Werte im Hinblick auf die Verabreichung von IgG können denen der Sofortprophylaxe von viralen Infektionen entsprechen: Die Dosen liegen deutlich tiefer, z.B. 10 bis 20 mg/kg Körpergewicht für Hepatitis A, und 50 mg/kg Körpergewicht bei Masern und Varicellen.

Nach dem Stand der Untersuchungen ist es bevorzugt eine Mindestdosis von 100 mg/kg Körpergewicht und als besonders bevorzugte Dosis das 2- bis 5-fache davon, nämlich 200 bis 500 mg/kg Körpergewicht zu verabreichnen.

Es ist bevorzugt, dass die Antikörper in der erfindungsgemässen pharmazeutischen Zusammensetzung in einer hohen Konzentration enthalten sind, so dass eine hochdosierte Verabreichung ermöglicht wird.

Im Hinblick auf ein bevorzugtes Verhältnis von Virus zu Antikörper in der erfindungsgemässen pharmazeutischen Zusammensetzung wird man bestrebt sein, ein Vielfaches der Menge an Antikörper zur Neutralisierung des Virus einzusetzen, um dem Sicherheitsbestreben voll Rechnung zu tragen. Dabei darf aber die Antikörperdosis nicht so hoch sein, dass die Zielsetzung, nämlich die Immunisierung, verhindert wird.

Die erfindungsgemäss durchgeführten Tierversuche an Schimpansen haben deutlich gezeigt, dass bei Verabreichung der beanspruchten pharmazeutischen Präparation eine Mitigierung des Verlaufs der Erkrankung nach einer Infektion mit dem Virus erzielt werden kann. Die erfindungsgemässen Präparationen führen zu einer Verbesserung der Leberhistologie und damit zu einer Verbesserung der Leberfunktion eines mit Virus infizierten Patienten.

Die vorliegende Erfindung beschränkt sich nicht etwa auf eine Mitigierung des Krankheitsverlaufes nach einer HCV-Infektion, sondern betrifft insgesamt pharmazeutische Zusammensetzungen, die geeignet sind, bei Virusinfektionen allgemein eine verstärkte-Immunität des Wirts gegenüber dem Krankheitserreger hervorzurufen. Insbesondere eignen sich die erfindungsgemässen pharmazeutischen Präparationen als Impfstoffe gegen Viren, die durch häufige Mutationen ihr Erscheinungbild ständig verändern.

Eine limitierte Protektion kann vor allem bei chronischen Erkrankungen von Vorteil sein. Jedenfalls wurde beobachtet, daß eine erfindungsgemäße pharmazeutische Zusammensetzung in einem Schimpansen nicht zu einem vollständigen Schutz vor einer Erkrankung an Hepatitis C führte, jedoch die Erkrankung des Schimpansen nicht so schwer wie erwartet war. Dabei wurde beobachtet, daß die Leberfunktionswerte gegenüber einem unbehandelten Schimpansen verbessert waren. Es kam zu keiner Erhöhung des leberspezifischen Enzymes ALT (Amino-alaninetransferase) im Serum im Gegensatz zum unbehandelten Schimpansen. Von hepatotropen Viren ist bekannt, daß diese bei Infektion in Abwesenheit von protektiven Antikörpern zu Leberfunktionsveränderungen im Sinne einer Erhöhung der Leberenzyme im Serum (ALT = Amino-alanin-transferase, AST = Amino-aspartat-transferase) führen. Die Erhöhung des Leberenzymes ALT ist im allgemeinen Ausdruck der Nekrose der Hepatozyten. Bei chronischen Infektionen kann es zu monatelangen bis jahrelangen Schädigungen der Hepatozyten durch das Hepatitisvirus und zum Persistieren der erhöhten Serum-Leberenzyme kommen.

In der Folge kann es auch zu einem Umbau des Lebergewebes kommen, wobei es auch zur Ausbildung einer Leberzirrhose und z. B. bei Hepatitis B zum Auftreten eines Leberkarzinoms kommen kann. Bei Hepatitis C kann diese Phase mit leichter Erhöhung der ALT-Werte einhergehen, aber die Chronizität ist bedeutend höher als bei Hepatitis B. Ob Hepatitis C als eine der Ursachen des Leberkarzinoms eine bedeutende Rolle spielt, ist noch nicht entschieden. Das Ausmaß der Chronizität von Hepatitiden könnte auch vom Grad der Mutationsrate dieser Viren bei fehlender Antikörperbildung abhängen bzw. wie von FARCI et al. beschrieben, durch die protrahierte Bildung spezifischer Antikörper verursacht sein. Die Chronizität kann auch durch Reinfektion mit anderen Geno- oder Subtypen des gleichen Virus ausgelöst werden. Eine limitierte Infektion und/oder die Verhinderung der Chronizität ist bereits als Erfolg anzusehen.

Die Erfindung umfasst ausserdem ein Set zur Herstellung von pharmazeutischen Zusammensetzungen, wobei dieses Set (a) das gereinigte Virus bzw. das in einer Fraktion angereicherte Virus und (b) die das Virus neutralisierenden Antikörper umfasst.

Im weiteren umfasst die Erfindung die Verwendung eines mit Antikörper neutralisierten Virus zur Herstellung einer pharmazeutischen Präparation, welche zur Mitigierung des Verlaufs einer Erkrankung nach einer Infektion mit dem Virus geeignet ist. Insbesondere wird erfindungsgemäss eine Verbesserung der Leberhistologie erzielt, was praktisch eine Verbesserung der Leberfunktion des Patienten bedeutet.

Anhand des folgenden Beispiels soll die Erfindung näher erläutert werden.

Das folgende Beispiel erläutert den Versuch bei hepatotropen Viren. Bei anderen Viren muss das entsprechende Zielorgan untersucht und beurteilt werden.

### Material und Methoden:

### 1. Versuchstiere:

Der Schimpanse ist das geeignete Tiermodell, um die protektive Wirkung einer prophylaktischen Therapie mit der erfindungsgemässen Pharmazeutischen Zusammensetzung zu prüfen (Tabor et al, J. Med. Primatol. 12: 305-318, (1983)). Für manche Viren können jedoch auch andere Primaten verwendet werden (z.B. Cynomolgusaffen für Hepatitis E).

Acht naive Schimpansen (pan troglodytes) werden für diesen Versuch eingesetzt. Die Schimpansen müssen völlig gesund sein, dürfen keine Marker der Hepatitis B, C und D aufweisen, und dürfen in keinem Versuch mit humanem Material, z.B. Faktor VIII-Konzentrat oder Vaccine gegen das in der pharmazeutischen Zusammensetzung (abgekürzt PZ) verwendete Virus, gewesen sein. Die Schimpansen sollen vorzugsweise in Tierräumen, die einer Biosicherheitsstufe 2 entsprechen, gehalten werden. Zwei der acht Schimpansen sind Kontrolltiere und erhalten nicht die PZ. Die anderen Schimpansen erhalten entweder im Abstand von 4 Wochen oder 6 Monaten mehrmals intravenös, intramuskulär oder sub- oder intrakutan die PZ (evtl. je 2 Schimpansen). Sämtliche Blutabnahmen und Leberbiopsien werden unter Ketamin-Anaestesie durchgeführt.

### 2. Hepatitis-Serologie:

Die Schimpansen werden in einer Vorphase zum Versuch, der mindestens 4 Monate dauert, auf Hepatitis B-, C- und D-Marker untersucht. Folgende Tests müssen nicht nur wegen des zu erwartenden Virus, sondern wegen einer möglichen Interferenz negativ sein:
Hepatitis B-Oberflächen-Antigen
Hepatitis B-Oberflächen-Antikörper
Hepatitis B-Kern-Antikörper (IgG und IgM)
Hepatitis B-Antigen und -Antikörper
HBV DNA mittels PCR
Hepatitis C-Antikörper
Hepatitis C-Riba-Test
HCV RNA mittels PCR
Hepatitis D-Antikörper
Hepatitis D-Antigen.

Sollen andere Präparationen, z.B. gegen Hepatitis E, ausgetestet werden, müssen auch sämtliche Marker gegen dieses Virus - Hepatitis E - negativ sein. Sämtliche Tests müssen einer internen und wenn möglich externen Qualitätskontrolle unterworfen werden. Alle Tests werden in zweiwöchentlichem Abstand durchgeführt.

### 3. Leberenzyme:

Zur Prüfung der Leberfunktion bzw. als Indikator einer akuten Entzündung werden die beiden Enzyme Alaninaminotransferase (ALT EC No. 2.6.1.2) und Gamma-glutamyltransferase (G-GT EC No. 2.3.2.2.) verwendet. Die Bestimmung erfolgt im Serum der wöchentlichen Blutabnahmen. Gesunde Schimpansen haben im Serum eine ALT-Konzentration, die dem des Menschen entspricht (Eder et al, The Role of the Chimpanzee in Research, Symp., Wien, 156-165, (1994)). Die Bestimmung erfolgt gemäss den Empfehlungen der 'Deutschen Gesellschaft für Klinische Chemie' (1972). Der ALT-Wert im Serum ist erhöht, wenn es zu einer Schädigung der Hepatozyten infolge erhöhter Permeabilität der Zellmembran oder zu einer Zellnekrose kommt (Thefield et al, Dtsch.med.Wschr. 994: 343-351 (1974), Wallnoefer et al, in Synopsis der Leberkrankheiten, Thieme Verlag, Stuttgart (1974), Schmidt et al, Laboratory and Clinical Science, New York, 411-487 (1983), Wuest H, Klin. Gastroenterologie in 2 Bänden, Thieme Verlag Stuttgart, (1984)). Es ist bekannt, dass in 70 bis 100% der Fälle bei Inokulierung mit Hepatitis B-Virus beim Schimpansen eine deutliche Erhöhung der ALT-Werte auftritt (Tabor et al 1983). Vor Entdeckung des Hepatitis C-Virus wurde eine ALT-Erhöhung über das 2,5-fache des oberen Normalwertes als indikativ für eine Hepatitis C betrachtet. Auch bei Hepatitis, welche durch andere Viren ausgelöst wird, wird eine Erhöhung der ALT gefunden (Eder, ALT- und G-GT-Erhöhung bei EBV-Hepatitis des Schimpansen, unveröffentlichte Ergebnisse). Das Enzym G-GT hingegen ist hauptsächlich in der Membran jener Zellen, die eine hohe absorptive oder sekretorische Aufgabe zu erfüllen haben, nachweisbar (nämlich Leber, Niere, Pancreas). Die biologische Rolle der G-GT ist noch nicht völlig geklärt. Im Gegensatz zum Enzym ALT ist G-GT Membran-gebunden und in den Gallengangs- und Kupffer-Zellen nachweisbar (Rosalik et al, Clin. Chem. Acta 39: 41-47 (1972), Rosalik et al, Clin.Chem. 7:53-107 (1975); Valenza et al, J.Med. Primatol. 14:350-315 (1985); Weill et al, Gammaglutamyltransferases: advances in biomedical pharmacology, 3.Serie, Masson, Paris, S. 195-198 (1982)). Nur in einem kleinen Prozentsatz wird G-GT in den Parenchymzellen gefunden. Da G-GT aktiv in das Serum sezerniert wird, ist dieses Enzym der sensitivste Indikator für eine Beschädigung der Zellmembran und kann erfolgreich zum Nachweis einer Hepatitis verwendet werden. Bei einer Erhöhung der G-GT muss eine Obstruktion der Gallenwege und Alkoholaufnahme bzw. Missbrauch ausgeschlossen werden.

Eigene, unpublizierte Untersuchungen haben gezeigt, dass G-GT ein besserer Indikator als ALT für den Nachweis einer Leberentzündung ist. Die Bestimmung erfolgt entsprechend den Empfehlungen der Deutschen Gesellschaft für Klinische Chemie (Persijn et al, J. Clin. Chem. Clin. Biochem. 149, 421-427 (1976)). Die Normalwerte des Schimpansen entsprechen dem des Menschen.

### 4. Untersuchung des Lebergewebes:

Die Tiere werden in der Vor- und Versuchsphase im Abstand von 2 Wochen Leber-punktiert. Verwendet wird eine Leberbioposienadel nach Menghini oder ein anderes kommerziell erhältliches System. Die entnommenen Lebergewebeproben werden für die lichtmikroskopische Untersuchung in Paraformaldehyd fixiert, für die immunhistochemische Untersuchung in flüssigem Stickstoff schockgefroren und für die elektromikroskopische Untersuchung in Glutaraldehyd fixiert. Sämtliche Untersuchungen werden in kodifizierter Form durchgeführt. Die für die Lichtmikroskopie vorgesehenen Proben werden routinemässig mit Haematoxyllin Eosin, PAS und Mallory gefärbt. Immunhistochemisch werden polyklonale und monoklonale Antikörper, die mit einem oder mehreren Antigenen des Virus reagieren, verwendet. Die Antikörper werden entweder mit Fluoreszin oder Biotin konjugiert.

Alle Leberbiopsiebefunde der Schimpansen müssen in der Vorphase normal sein. Eine geringgradige, nicht spezifische reaktive Hepatitis kann toleriert werden, da diese immer wieder auch bei völlig gesunden Schimpansen gefunden wird. Die Befunde während der Versuchsphase werden nach dem Humanem Score System (Skala 0 bis 18) graduiert (Ishak et al, J. Hepatol. 22:696-699 (1985)). In den elektromikroskopischen Untersuchungen werden insbesondere die ultrastrukturellen Veränderungen, wie sie für virale Infektionen, z.B. Hepatitis C, beschrieben sind, beurteilt (Pfeifer et al, Virchow Archiv B Cell Pathol. 20:617-627 (1980); Schaff et al, Virchow Arch. B Cell Pathol. 45: 301-312 (1984), Schaff et al, J. Ultrastr. Path. 14:303-309 (1990)).

### 5. Pharmazeutische Zusammensetzung:

Die PZ enthält bereits die Immunglobulin-Präparation oder diese wird getrennt zur Zubereitung, die das nicht zu einer produktiven Infektion führende Virus enthält, verabreicht.

Bei den Versuchen zu Hepatitis C kann die Virus-Präparation mehrere Genotypen sowie ein Gemisch aus mehreren Subtypen umfassen.

Die eingesetzte Virusmenge entspricht einer Konzentration eines produktiven Virus von 10³ CID 50/ml. Die Gammaglobulin-Gabe, die intravenös verabreicht wird, beträgt 100 mg/kg Körpergewicht.

### Versuchsdurchführung:

Die beiden Kontrolltiere erhalten keine pharmazeutische Zubereitung. Alle Tiere werden nach Erreichen entsprechender Antikörperbildung bei den Versuchstieren einem Challenge-Versuch unterworfen. Die Challenge-Dosis entspricht einer mittleren Konzentration an produktivem Virus, wie es im Krankheitsfall gefunden wird, z.B. Hepatitis C 10^{1,5} CID/ml.

### Ergebnisse:

Bei den beiden Kontrollschimpansen, die keine pharmazeutische Zubereitung erhielten, lassen sich eine Virusinfektion nach entsprechender Inkubationszeit, eine Erhöhung der G-GT mit und ohne ALT-Erhöhung sowie virale Genom-Äquivalente nach dem Challenge nachweisen. Dies demonstriert die Wirksamkeit des Challenge-Materials.

Bei den übrigen Versuchstieren lässt sich nach Gabe der PZ der im Überschuss vorhandene, passiv zugeführte Antikörper entsprechend seiner Halbwertszeit nachweisen. Nach dem Challenge zeigt jener Schimpanse, der die geringste Dosis der PZ erhielt, die längste Halbwertszeit des zugeführten viralen Antigens des Challenge-Materials.

Bei den immunisierten Schimpansen lassen sich elektronenmikroskopisch keine ultrastrukturellen Veränderungen im Sinne einer viralen Infektion der Leber und lichtmikroskopisch keine akute Hepatitis nachweisen. Es kommt post Challenge zu keiner Erhöhung der Leberenzyme ALT und G-GT; es lässt sich weder Virus aus Serum oder Lebergewebe isolieren, noch konsekutiv mittels PCR nachweisen.

## Patentansprüche

1. Pharmazeutische Zusammensetzung zur Prophylaxe oder Therapie von Viruserkrankungen, die verursacht sind durch Viren hoher Mutationsrate, dadurch gekennzeichnet, dass diese ein gereinigtes, inaktiviertes Virus und für dieses spezifische neutralisierende Antikörper enthält.

2. Pharmazeutische Zusammensetzung nach Anspruch 1, dadurch gekennzeichnet, dass das Virus im Konjugat *in vivo* nicht zur einer produktiven Infektion führt.

3. Pharmazeutische Zusammensetzung nach einem der Ansprüche 1 oder 2, dadurch gekennzeichnet, dass das Virus ausgewählt ist aus der Gruppe der Flaviviren, der Picornaviren, der Hepatitisviren, der Retroviren, der Pestiviren und der Parvoviren.

4. Pharmazeutische Zusammensetzung nach einem der Ansprüche 1 bis 3, dadurch gekennzeichnet, dass das Virus ein Hepatitis C-Virus ist.

5. Pharmazeutische Zusammensetzung nach einem der Ansprüche 1 bis 4, dadurch gekennzeichnet, dass sie ein Virus mehrerer Genotypen und/oder ein Gemisch aus mehreren Subtypen enthält.

6. Pharmazeutische Zusammensetzung nach einem der Ansprüche 1 bis 5, dadurch gekennzeichnet, dass sie ein Fragment eines gereinigten Virus enthält.

7. Pharmazeutische Zusammensetzung nach einem der Ansprüche 1 bis 6, dadurch gekennzeichnet, dass die neutralisierenden Antikörper protektive Antikörper sind.

8. Pharmazeutische Zusammensetzung nach einem der Ansprüche 1 bis 7, dadurch gekennzeichnet, dass die Antikörper aus Plasma oder einer Plasmafraktion stammen.

9. Pharmazeutische Zusammensetzung nach einem der Ansprüche 1 bis 8, dadurch gekennzeichnet, dass die Antikörper aus einer Fraktion stammen, die zur Inaktivierung oder Abreicherung von möglicherweise vorhandenen Viren behandelt ist.

10. Pharmazeutische Zusammensetzung nach einem der Ansprüche 1 bis 9, dadurch gekennzeichnet, dass die Antikörper gegen mehr als ein Epitop gerichtet sind.

11. Pharmazeutische Zusammensetzung nach einem der Ansprüche 1 bis 10, dadurch gekennzeichnet, dass die Antikörper gegen mehr als ein virales Antigen gerichtet sind.

12. Pharmazeutische Zusammensetzung nach einem der Ansprüche 1 bis 11, dadurch gekennzeichnet, dass die Antikörper gegen ein Kernprotein und/oder Hüllprotein des Virus gerichtet sind.

13. Pharmazeutische Zusammensetzung nach Anspruch 1 bis 12, dadurch gekennzeichnet, dass die Antikörper in einer Konzentration enthalten sind, die eine Dosis von 100 bis 1000 mg/kg Körpergewicht ermöglicht.

14. Pharmazeutische Zusammensetzung nach einem der Ansprüche 1 bis 13, dadurch gekennzeichnet, dass die Zusammensetzung zur Inaktivierung von Viren chemisch oder physikalisch vorbehandelt ist.

15. Pharmazeutische Zusammensetzung nach einem der Ansprüche 1 bis 14, dadurch gekennzeichnet, dass sie Komplement enthält.

16. Pharmazeutische Zusammensetzung nach einem oder mehreren der Ansprüche 1 bis 15, dadurch gekennzeichnet, dass sie zur intravenösen Verabreichung geeignet ist.

17. Pharmazeutische Zusammensetzung nach einem der Ansprüche 1 bis 15, dadurch gekennzeichnet, dass sie zur mukosalen Verabreichung geeignet ist.

18. Set zur Herstellung einer pharmazeutischen Zusammensetzung gemäß einem der Ansprüche 1 bis 17, dadurch gekennzeichnet, dass dieses umfasst:
a) das gereinigte Virus, und
b) die das Virus neutralisierende Antikörper.

19. Verwendung einer pharmazeutischen Zusammensetzung nach einem der Ansprüche 1 bis 17 zur Herstellung einer pharmazeutischen Präparation zur Mitigierung des Verlaufs einer Erkrankung nach einer Infektion.

20. Verwendung nach Anspruch 19 zur Verbesserung der Leberhistologie und Leberfunktion.

## Claims

1. Pharmaceutical composition for the prophylaxis or treatment of viral diseases which are caused by viruses of high mutation rate, characterised in that it contains a purified, inactivated virus and specific neutralising antibodies for the latter.

2. Pharmaceutical composition according to claim 1, characterised in that the virus in the conjugate *in vivo* does not lead to a productive infection.

3. Pharmaceutical composition according to one of claims 1 or 2, characterised in that the virus is selected from the group comprising flavoviruses, picornaviruses, hepatitis viruses, retroviruses, pestiviruses and parvoviruses.

4. Pharmaceutical composition according to one of claims 1 to 3, characterised in that the virus is a hepatitis C virus.

5. Pharmaceutical composition according to one of claims 1 to 4, characterised in that it contains a virus of several genotypes and/or a mixture of several sub-types.

6. Pharmaceutical composition according to one of claims 1 to 5, characterised in that it contains a fragment of a purified virus.

7. Pharmaceutical composition according to one of claims 1 to 6, characterised in that the neutralising antibodies are protective antibodies.

8. Pharmaceutical composition according to one of claims 1 to 7, characterised in that the antibodies originate from plasma or a plasma fraction.

9. Pharmaceutical composition according to one of claims 1 to 8, characterised in that the antibodies originate from a fraction which is treated to inactivate or deplete viruses which are possibly present.

10. Pharmaceutical composition according to one of claims 1 to 9, characterised in that the antibodies are directed to more than one epitope.

11. Pharmaceutical composition according to one of claims 1 to 10, characterised in that the antibodies are directed to more than one viral antigen.

12. Pharmaceutical composition according to one of claims 1 to 11, characterised in that the antibodies are directed to one core protein and/or coat protein of the virus.

13. Pharmaceutical composition according to claim 1 to 12, characterised in that the antibodies are present in a concentration which facilitates a dose of 100 to 1,000 mg/kg of body weight.

14. Pharmaceutical composition according to one of claims 1 to 13, characterised in that the composition is pretreated chemically or physically to inactivate viruses.

15. Pharmaceutical composition according to one of claims 1 to 14, characterised in that it contains complement.

16. Pharmaceutical composition according to one or more of claims 1 to 15, characterised in that it is suitable for intravenous administration.

17. Pharmaceutical composition according to one of claims 1 to 15, characterised in that it is suitable for mucosal administration.

18. Set for producing a pharmaceutical composition according to one of claims 1 to 17, characterised in that it comprises:
a) the purified virus, and
b) the antibodies neutralising the virus.

19. Use of a pharmaceutical composition according to one of claims 1 to 17 for producing a pharmaceutical preparation for mitigation of the course of a disease after infection.

20. Use according to claim 19 for improving the liver histology and liver function.

## Revendications

1. Composition pharmaceutique pour la prophylaxie ou la thérapie de maladies virales qui sont provoquées par des virus à fort taux de mutation, caractérisée en ce qu'elle contient un virus purifié et inactivé, et des anticorps spécifiques qui neutralisent ce dernier.

2. Composition pharmaceutique selon la revendication 1, caractérisée en ce que le virus n'entraîne pas une infection productive dans le conjugué in vivo.

3. Composition pharmaceutique selon l'une des revendications 1 et 2, caractérisée en ce que le virus est sélectionné dans le groupe des flavivirus, des picornavirus, des virus de l'hépatite, des rétrovirus, des pestivirus et des parvovirus.

4. Composition pharmaceutique selon l'une des revendications 1 à 3, caractérisée en ce que le virus est un virus de l'hépatite C.

5. Composition pharmaceutique selon l'une des revendications 1 à 4, caractérisée en ce qu'elle contient un virus de plusieurs génotypes et/ou un mélange de plusieurs sous-types.

6. Composition pharmaceutique selon l'une des revendications 1 à 5, caractérisée en ce qu'elle contient un fragment d'un virus purifié.

7. Composition pharmaceutique selon l'une des revendications 1 à 6, caractérisée en ce que les anticorps neutralisants sont des anticorps protecteurs.

8. Composition pharmaceutique selon l'une des revendications 1 à 7, caractérisée en ce que les anticorps proviennent de plasma ou d'une fraction de plasma.

9. Composition pharmaceutique selon l'une des revendications 1 à 8, caractérisée en ce que les anticorps proviennent d'une fraction qui est traitée en vue de l'inactivation ou de l'appauvrissement de virus éventuellement présents.

10. Composition pharmaceutique selon l'une des revendications 1 à 9, caractérisée en ce que les anticorps sont spécifiques de plus d'un épitope.

11. Composition pharmaceutique selon l'une des revendications 1 à 10, caractérisée en ce que les anticorps sont spécifiques de plus d'un antigène viral.

12. Composition pharmaceutique selon l'une des revendications 1 à 11, caractérisée en ce que les anticorps sont spécifiques d'une protéine interne et/ou d'une protéine d'enveloppe du virus.

13. Composition pharmaceutique selon les revendications 1 à 12, caractérisée en ce que les anticorps sont contenus à une concentration qui permet un dosage de 100 à 1 000 mg/kg de poids corporel.

14. Composition pharmaceutique selon l'une des revendications 1 à 13, caractérisée en ce que la composition est traitée chimiquement ou physiquement en vue de l'inactivation de virus.

15. Composition pharmaceutique selon l'une des revendications 1 à 14, caractérisée en ce qu'elle contient un complément.

16. Composition pharmaceutique selon l'une ou plusieurs des revendications 1 à 15, caractérisée en ce qu'elle convient pour être administrée par voie intraveineuse.

17. Composition pharmaceutique selon l'une des revendications 1 à 15, caractérisée en ce qu'elle convient pour être administrée par les muqueuses.

18. Ensemble pour la préparation d'une composition pharmaceutique selon l'une des revendications 1 à 17, caractérisé en ce qu'il comprend:
a) le virus purifié, et
b) l'anticorps qui neutralise le virus.

19. Utilisation d'une composition pharmaceutique selon l'une des revendications 1 à 17, pour la préparation d'une préparation pharmaceutique destinée à adoucir l'évolution d'une maladie après une infection.

20. Utilisation selon la revendication 19, pour l'amélioration de l'histologie du foie et du fonctionnement du foie.
